# EUROPEAN PATENT SPECIFICATION

(11) **EP 0 957 790 B1**
(45) Date of publication and mention of the grant of the patent: **17.12.2003**
(21) Application number: 96911060.0
(22) Date of filing: 26.04.1996
(51) Int. Cl.: A61B 18/20, A61C 1/00

(54) **LASER SYSTEM**
LASERSYSTEM
SYSTEME LASER

(30) Priority: 04.05.1995 GB 9509126; 15.02.1996 GB 9603143
(43) Date of publication of application: 24.11.1999
(73) Proprietor: Opusdent Ltd., Netanya 42505 (IL)
(72) Inventor: COLLES, Michael, John, Hartree By Biggar, Lanarkshire ML12 6TG (GB)
(74) Representative: Gold, Tibor Z.
(86) International application number: GB9601002
(87) International publication number: WO96034566

(56) References cited:
- EP-A- 0 256 671
- WO-A-94/26203
- US-A- 4 638 800
- US-A- 5 207 671
- US-A- 5 321 715

## Description

The present invention relates to the application of lasers for the controlled ablation of tissue. More particularly the invention relates to the application of lasers in dentistry.

In this field it is known that certain lasers can be used to drill through hard biological tissue such as bone and the tooth enamel and dentine of teeth. In particular the Erbium:YAG laser operating at a wavelength of 2.94 µm, a wavelength that is especially strongly absorbed by water bearing tissue, has demonstrated efficient drilling without substantial damage to surrounding tissue when used in conjunction with a water spray. Such systems are now becoming available commercially. Short wavelength (0.19µm) Excimer lasers show a similar ability to provide clean cuts in tooth and bone tissue. These lasers provide relatively high energy pulses at a low repetition rate. Carbon dioxide lasers configured to operate in a similar way, that is with high energy short pulses (<10µs) at a low rate have also been reported to offer reasonably clean drilling although it is perceived that less strong water absorption at their wavelength of 10.6µm renders them less effective. It has been demonstrated that the far more common continuous wave (cw) or so-called "superpulse" CO₂ lasers overheat teeth with both undesirable carbon formation and potential damage to any underlying vital pulp.

These approaches have a number of disadvantages in terms of their application to dentistry. In particular the Excimer lasers are large and expensive requiring careful chemical engineering in the handling of their toxic gas constituents. This makes them entirely inappropriate for typical dental installations. Similarly the short pulse high energy CO₂ lasers, whilst common in industrial settings have no history of use in medical applications. The Erbium:YAG laser clearly can drill teeth effectively although it also is a relatively expensive system in the context of a typical dental installation.

The present invention aims to overcome the aforementioned problems of the prior art.

According to the present invention there is provided a CO₂ laser system for ablation of hard tissue comprising a laser head and control means adapted to produce a focused laser beam in pulses from the laser head to an area of hard tissue to be ablated, characterised in that the control means is adapted to produce the focused laser beam in organised bursts of pulses, each burst comprising between 2 and 10 pulses, wherein each pulse has a first duration (Tₚ) of between 100 and 700µs and is separated from the next pulse in the burst by a period of time having a second duration (T_{g}) of between 200 and 1000µsec, the bursts having a burst repeat frequency of between 10 and 100Hz.

Preferably the system further comprising scanning means by which the focused beam traverses a defined area such as an area of tissue to be ablated.

Preferably the wavelength (λ) of the laser pulses is between 9 and 11µm.

Preferably said second duration is between 250 and 600µs.

Preferably said second duration is between 250 and 350µs.

Preferably the burst repeat frequency is between 30 and 50Hz.

Preferably 10 to 50mJ of pulse energy is delivered in a beam focused to a diameter of typically 300µm.

As described herein a method of effective drilling of all types of tooth tissue can be achieved with the output beam from suitably modified CO₂ lasers typical of those used commonly in medical applications.

To achieve clean drilling without either damage to adjacent tissue or subsequent cracking of enamel and dentine requires a specific organisation of the output into groups of pulses as described below. It is this specific grouping of pulses together with their duration and profile which constitutes the successful activity of this invention. The relatively low energy pulses from such quasi-cw lasers can still produce efficient explosive ablation provided that the beam is focused down to a spot size of sufficiently small size. The invention may further provide a scanning means by which a small spot can traverse in an automatic preset fashion a pattern that gives the size of the drill hole characteristically required in dentistry. The timing of the grouped laser pulses on the scanned spot is achieved in such a way that a simple drilling action is perceived. This combination of specific arrangements of pulses in an output beam that is tightly focused and scanned provides a novel and non-obvious route to laser drilling of teeth using CO₂ lasers. The process as with current Er:YAG systems or standard mechanical drills requires the simultaneous presence of a fine water spray to assist in cooling.

Specifically it can be demonstrated that the following of features lead to the beneficial removal of dental hard tissue:
1. Pulsing of the laser for significantly lower durations than in the established "superpulse" mode.
2. Limiting the output to a burst of such pulses relatively few in number.
3. Providing a long enough gap between such bursts such that their advantageous characteristics are not affected.
4. Ensuring a strong interaction by tightly focusing the high quality beam onto the tissue.
5. Ensuring that the surfaces are maintained wet to further enhance the interaction and provide additional cooling.

In support of these individual aspects it can be argued that reducing the laser pulse duration to, for example 200µs enhances the relative contribution of the initiating spike to the overall pulse energy. The contribution from this spike is in any case greater in the first few pulses from an initial turn on due to the lack of ionised particles in the laser gas and the consequent need for more energy to initiate the discharge. By limiting the number of pulses delivered to a low number within a burst and further limiting the burst frequency such that each can be regarded as seeing an electrically cold gas, such an enhancement of the pulse power can be maintained. The level of enhancement depends on the position of a pulse within a burst; the first carrying a significant proportion of its energy within the short initiating spike (a few µs duration) and thus peak powers typically in excess of 200 watts, (in a laser giving around 20 watts when used cw), whilst subsequent pulses typically carry an increasing, but more uniformly distributed, energy. Overall the average power enhancement factor is typically x5 compared with the normal superpulse mode enhancement of x2.

The initial spike provides the high power which makes this mode of operation similar in its effectiveness on hard tissue to the previously mentioned purely pulsed CO₂ systems having durations of less than 10µs. Having initiated the interaction on tissue subsequent pulses within the burst provides the energy needed to remove significant volumes of material. In order to provide an effective drilling the burst must be repeated at a sufficiently high rate to give significant material removal. This must be balanced by both the requirements of maintaining the characteristics of an individual burst and allowing the tissue immediately adjacent to the ablated region to cool.

The requirement for the successful ablation of any tissue is to supply efficient energy at a high enough rate to ensure removal of the affected zone before. conduction processes have led to any potentially damaging heating of the surrounding area. This is achieved in dental hard tissues by using very high power densities, typically 50kW/cm². In the case of our example here of a relatively low power cw equivalent laser this implies focusing to small spot size of around 0.3mm diameter. The appearance of a larger drill hole, as required to match conventional dental practice, together with further interpulse cooling of a given interaction volume is achieved by scanning the beam in for example a circular fashion such that an overall diameter of around 1mm is achieved. The gap between subsequent pulses over the same position is consequently increased to 4 times the interpulse spacing. (Such scanning is materially different to that introduced by US Patent 5411502 wherein the interaction mechanism itself is specifically controlled by the rapid scanning of a cw beam. The scanning referred to in the present invention has no effect on the interactive mechanism which results from the nature of the pulse sequences and the tightly focussed beam).

The water content of both enamel and dentine is significantly less than that of soft tissue. The water spray enhances the surface water in the region of the impact area and thus assists the absorption of the laser energy. It also provides an additional and important heat sink which is effective in ensuring both virtually no cracking to the surrounding enamel and no significant temperature rise within the underlying tissue.

A CO₂ laser set to operate within this combination of parameters defined herein satisfactorily drills enamel and dentine without cracking or pulpal heating. Experimentally this has been demonstrated by varying the pulse duration Tp, the gap between pulses in a burst Tg, the number of pulses in a burst Nb and the burst repetition frequency to identify an optimum combination of parameters for both enamel and dentine. Whilst an optimum set can be identified an effective and safe interaction can be achieved over a reasonable range of each individual parameter. For example enamel is best addressed with a lower Nb and shorter Tp. Softer material such as carious dentine is less demanding and can tolerate more and longer pulses in each burst.

This has been determined that pulse durations (Tp) from 100µs to 700µs are effective in removing hard tissue but preferably within the range 200 to 500µs. These parameters have to be offered in a group or burst of pulses. This burst provides clean removal of tissue at a significant rate given that the pulse separation is between 200 and 1000µs but is preferably between 250 and 600µs and most preferably 250 to 350µs. The preferable number of pulses within a burst lies between 1 and 10 most preferably between 1 and 5 for enamel removal and 2 and 10 for dentine removal.

The repetition rate of the bursts is within a range between 100Hz and 0, preferably between 75Hz and 10Hz and most preferably 50Hz to 30Hz.

This mode of operation of a CO₂ laser provides a materially different interaction to that achieved with a standard superpulse system (typically 600µs pulse durations at 500Hz rep rate). That the use of the combination of parameters described above enables a low power nominally cw CO₂ laser to drill enamel and that this is non-obvious in the light of accepted current perceptions. It has been noted that CO₂ laser operation of 9.6µm may be beneficial in the removal of hard tissue because of an enhanced absorption at that wavelength in hydroxyapatite, the principal inorganic matrix of enamel. Fowler, B.O. et al Arch. Oral. Biol. 1966; 11:477-492 infrared spectra of hydroxyapatites... Fowler, B.O. Inorganic Chem. 1973; 13:207 infrared studies of apatites. A laser operating at 9.6µm in a mode within the range of parameters described in this application may be effective.

The benefits of a system according to this application are in the provision of a lower cost laser drill and one which may be also configured to operate cw or superpulsed, within the same unit, to provide the well proved ability of CO₂ lasers to effectively cut soft tissue.

### Example

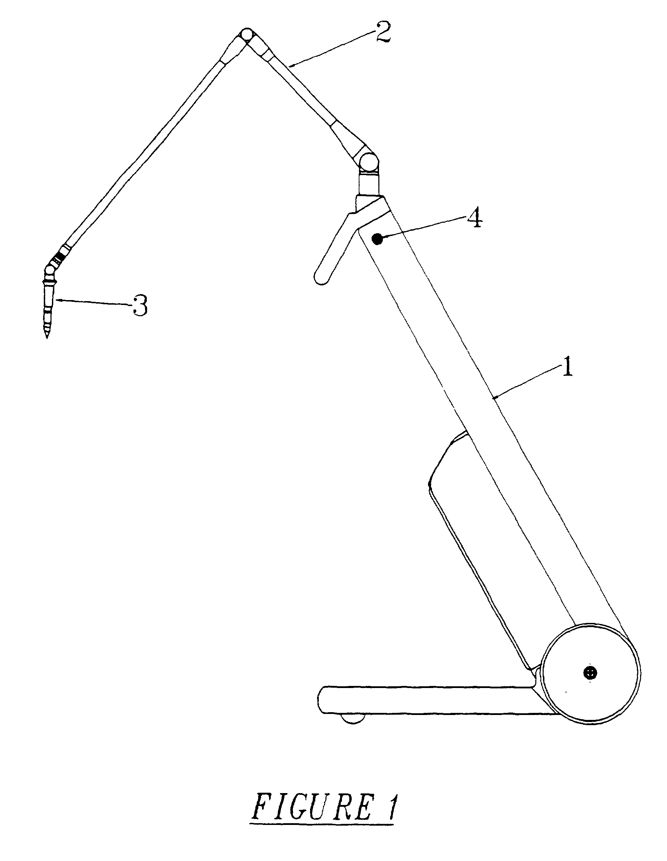

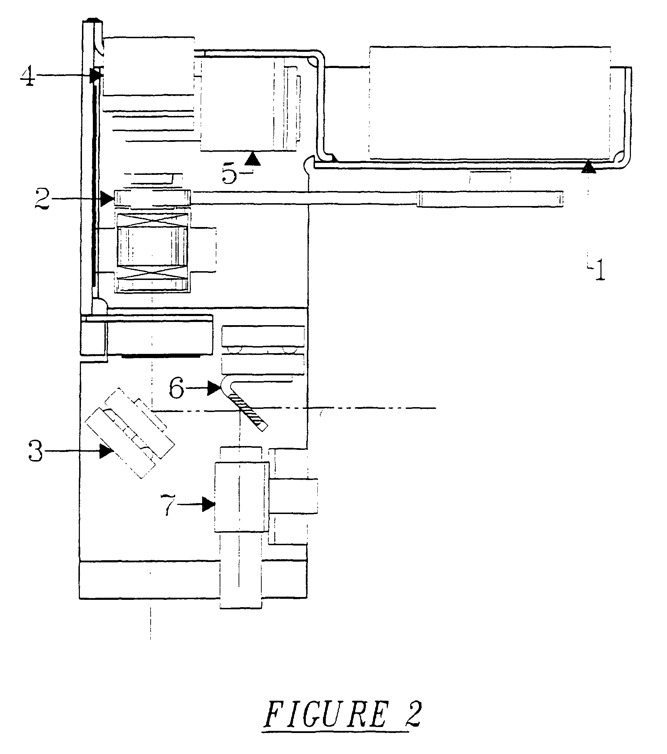

The pulse shape, grouping and frequency is controlled by electronic drives to the laser power supply. One embodiment of the system including laser head power supply and electronic controls is shown in figure 1. The main console 1 contains these components and a particular form of scanning arrangement, 4. The beam is conducted to the handpiece 3 which includes the water spray nozzle, via the articulated arm 2. The arm contains lensing such that the beam scanning pattern produced by the arrangement 4 in the console 1 is reproduced at the handpiece tip. Figure 2 shows one particular form of the scanning arrangement. The beam is conducted via deflectors 3, 4 and 5 to the articulated arm. In doing so the beam passes through the lens assembly 2 which is caused to rotate by gearing and a belt to stepper motor 1. If the rotational axis of the lens are not coincidental with the beam axis then the focal point describes a circle. The diameter of the circle may be adjusted by altering the amount by which the lens optic axis is displaced from the rotational and beam axis. (Other forms of scanning arrangements could be adopted including the use of vibrating mirrors and the scanning could take place elsewhere in the system, for example near the handpiece). A visible indicator beam is provided by a diode laser (7) combined coaxially with the invisible CO₂ laser beam using the combining optic (6).

A CO₂ laser drill operating at λ = 10.6µm delivers 250µs pulses with the number of pulses in a burst being selected by the user being between 1 and 8. The gap between pulses in a burst is 350µs with a burst frequency of 40Hz. In use for dental drilling 3 pulses per burst is recommended for drilling enamel, 7 for dentine or caries and 1 for clean up.

In summary the tailoring of the output of a CO₂ laser to provide groups of pulses having overall parameters within the bands specified above in a scanned tightly focused beam provides effective carbon free drilling of teeth.

The embodiment described above offers advantages in terms of simplicity and cost over existing systems.
In addition it offers significant clinical advantages in that, whilst set to provide typically encountered drill hole requirements, resetting to allow finer holes or cuts can be readily achieved through control of the scanner. Such fine cuts may find application in other disciplines and procedures for example in carrying out surgery on the microbones on the ear. A further advantage is that, in the same system, by returning to the conventional mode of operating the laser, the system can then be used for soft tissue surgery.

By changing the size of the scanned pattern a further application of the tailored pulse grouping would be to the fine surface ablation of epidermis as required in the procedure known as laser dermabrasion.

## Claims

1. A CO₂ laser system (1) for ablation of hard tissue comprising a laser head and control means adapted to produce a focused laser beam in pulses from the laser head to an area of hard tissue to be ablated, **characterised in that** the control means is adapted to produce the focused laser beam in organised bursts of pulses, each burst comprising between 2 and 10 pulses, wherein each pulse has a first duration (Tₚ) of between 100 and 700µs and is separated from the next pulse in the burst by a period of time having a second duration (T_{g}) of between 200 and 1000µsec, the bursts having a burst repeat frequency of between 10 and 100Hz.

2. A system as claimed in Claim 1, further comprising scanning means by which the focused beam traverses a defined area such as an area of tissue to be ablated.

3. A system as claimed in any preceding Claim wherein the wavelength (λ) of the laser pulses is between 9 and 11µm.

4. A system as claimed in any preceding Claim wherein said second duration is between 250 and 600µs.

5. A system as claimed in Claim 4 wherein said second duration is between 250 and 350µs.

6. A system as claimed in any preceding Claim wherein the burst repeat frequency is between 30 and 50Hz.

7. A system as claimed in any preceding Claim wherein 10 to 50mJ of pulse energy is delivered in a beam focused to a diameter of typically 300µm.

## Patentansprüche

1. Co₂-Lasersystem (1) zum Abtragen von Hartgewebe, wobei das System einen Laserkopf und Steuermittel aufweist, die zum Erzeugen eines fokussierten Laserstrahls in Impulsen vom Laserkopf zu einem Bereich von abzutragendem Hartgewebe geeignet sind, **dadurch gekennzeichnet, dass** das Steuermittel zum Erzeugen des fokussierten Laserstrahls in organisierten Ausstoßimpulsen geeignet ist, wobei jeder Ausstoß zwischen 2 und 10 Impulse aufweist, wobei jeder Impuls eine erste Dauer (Tₚ) zwischen 100 und 700 µs aufweist und vom nächsten Impuls im Ausstoß durch eine Zeitdauer getrennt ist, welche eine Zweite Dauer (T_{g}) von zwischen 200 und 1000 µSek aufweist, wobei die Ausstöße eine Ausstoßwiederholungsfrequenz von zwischen 10 und 100 Hz aufweisen.

2. System nach Anspruch 1, das ferner Abtastmittel aufweist, durch welche der fokussierte Strahl einen definierten Bereich wie einen Bereich von abzutragendem Gewebe durchquert.

3. System nach einem der vorhergehenden Ansprüche, wobei die Wellenlänge (λ) der Laserimpulse zwischen 9 und 11 µm liegt.

4. System nach einem der vorhergehenden Ansprüche, wobei die zweite Dauer zwischen 250 und 600 µs liegt.

5. System nach Anspruch 4, wobei die zweite Dauer zwischen 250 und 350 µs liegt.

6. System nach einem der vorhergehenden Ansprüche, wobei die Ausstoßwiederholungsfrequenz zwischen 30 und 50 Hz beträgt.

7. System nach einem der vorhergehenden Ansprüche, wobei 10 bis 50 mJ Impulsenergie in einem Strahl abgegeben werden, der auf einen Durchmesser von typischerweise 300 µm fokussiert ist.

## Revendications

1. Système à laser au CO₂ (1) pour l'ablation d'un tissu dur, comprenant une tête laser et un moyen de commande qui est à même de produire un faisceau laser focalisé sous forme d'impulsions issues de la tête laser sur une zone de tissu dur à ablater, **caractérisé en ce que** le moyen de commande est à même de produire le faisceau laser focalisé sous forme de rafales organisées d'impulsions, chaque rafale comprenant entre 2 et 10 impulsions, dans lequel chaque impulsion a une première durée (Tₚ) entre 100 et 700 µs et est séparée de l'impulsion suivante de la rafale d'une période de temps ayant une seconde durée (T_{g}) entre 200 et 1000 µs, les rafales ayant une fréquence de répétition entre 10 et 100 Hz.

2. Système selon la revendication 1, comprenant en outre un moyen de balayage qui fait passer le faisceau focalisé en travers d'une zone définie, telle qu'une zone de tissu à ablater.

3. Système selon l'une quelconque des revendications précédentes, dans lequel la longueur d'onde (λ) des impulsions laser se situe entre 9 et 11 µm.

4. Système selon l'une quelconque des revendications précédentes, dans lequel ladite seconde durée se situe entre 250 et 600 µs.

5. Système selon la revendication 4, dans lequel ladite seconde durée se situe entre 250 et 350 µs.

6. Système selon l'une quelconque des revendications précédentes, dans lequel la fréquence de répétition des rafales se situe entre 30 et 50 Hz.

7. Système selon l'une quelconque des revendications précédentes, dans lequel on délivre une énergie pulsée de 10 à 50 mJ dans un faisceau focalisé sur un diamètre typique de 300 µm.
